# EUROPÄISCHE PATENTANMELDUNG

(11) **EP 4 166 241 A1**
(43) Veröffentlichungstag der Anmeldung: **19.04.2023**
(21) Anmeldenummer: 22213242.5
(22) Anmeldetag: 08.10.2018
(51) Int. Cl.: B05B 12/00, B05B 11/00, B05B 11/10

(54) **SET AUS EINEM PUMPSPENDER UND EINER AUSWERTEEINHEIT**

(62) Teilanmeldung aus: 18199176.1
(71) Anmelder: Aptar Radolfzell GmbH, 78315 Radolfzell (DE)
(72) Erfinder: Graham, Dominic Alexander, Abingdon, Oxfordshire OX14 1XX (GB); Jones, Matthew Meredith, Warwick, Warwickshire CV34 6AU (GB); Körner, Joachim, 88690 Uhldingen-Mühlhofen (DE)
(74) Vertreter: Witte, Weller und Partner Patentanwälte mbB Stuttgart

(57) **Zusammenfassung**

Die Erfindung betrifft ein Set aus einem weitgehend herkömmlichen Pumpspender (10) zum Austrag von pharmazeutischen oder kosmetischen Flüssigkeiten und einer externen Auswerteeinheit (100), die wechselbar gestaltet ist, so dass sie nach Entleerung eines Pumpspenders (10) auf einen neuen Pumpspender (10) aufsetzbar ist.

Die Auswerteeinheit (100) kann so am Austragkopf (30) des Pumpspenders (10) angebracht sein, dass sie einen Betätigungsdrücker (60) erfasst und partiell gemeinsam mit diesem verlagerbar ist.

## Beschreibung

### ANWENDUNGSGEBIET UND STAND DER TECHNIK

Die Erfindung betrifft ein Set aus einem Pumpspender zum Austrag von pharmazeutischen oder kosmetischen Flüssigkeiten und einer Auswerteeinheit.

Pumpspender im Sinne der Erfindung sind dabei die aus dem Stand der Technik grundsätzlich bekannten Pumpspender mit einem Flüssigkeitsspeicher, an dessen oberen Ende ein Austragkopf vorgesehen ist. Dieser Austragkopf verfügt über eine Basis, die am Flüssigkeitsspeicher befestigt ist, sowie über einen Betätigungsdrücker, der zum Zwecke des Austrags gegenüber der Basis niederdrückbar ist. Der Austragkopf verfügt über eine Pumpeinrichtung, die durch Niederdrücken des Betätigungsdrückers betätigt werden kann und die bei Betätigung Flüssigkeit von einem Einlasskanal, der mit dem Flüssigkeitsspeicher verbunden ist, zu einer am Austragkopf vorgesehenen Austragöffnung fördert.

Pumpspender der beschriebenen Art sind vergleichsweise einfache Vorrichtungen, die preisgünstig hergestellt werden können. Es besteht der Wunsch, die Nutzung solcher Pumpspender zu protokollieren, um Mehrwerte sowohl für den Nutzer als auch für den Hersteller von Pumpspendern bzw. den darin enthaltenen Flüssigkeiten zu schaffen. Allerdings sind die elektronischen Komponenten zur Erfassung und Weiterverarbeitung von Betätigungen des Pumpspenders vergleichsweise teuer, so dass die Integration in Pumpspender in der Regel nicht wirtschaftlich wäre.

### AUFGABE UND LÖSUNG

Aufgabe der Erfindung ist es, einen Pumpspender der beschriebenen Art dahingehend weiterzubilden, dass ein Austrag erfasst wird, um datentechnisch weiterverarbeitet werden zu können.

Hierfür wird vorgeschlagen, ein Set aus einen Pumpspender der eingangs genannten Art und einer Auswerteeinheit zur Verfügung zu stellen, wobei die Auswerteeinheit zur Erfassung der Betätigung der Pumpeinrichtung ausgebildet ist.

Dabei ist der Grundgedanke der vorliegenden Erfindung, dass der Pumpspender um eine Auswerteeinheit ergänzt wird, die als externe Auswerteeinheit ausgebildet ist. Unter einer externen Auswerteeinheit im Sinne der Erfindung wird verstanden, dass diese für die Grundfunktionalität des Pumpspenders nicht erheblich ist. Der Pumpspender ist bei entfernter Auswerteeinheit separat funktionstauglich und kann in üblicher Weise verwendet werden. Die externe Auswerteeinheit des erfindungsgemäßen Sets ergänzt diesen Pumpspender und kann an der Außenseite des Pumpspenders derart angebracht werden, dass sie eine Betätigung durch Niederdrücken des Betätigungsdrückers erfassen kann. Sie ist in einfach lösbarer Weise am Pumpspender angebracht, so dass es dem Benutzer leicht fällt, den Pumpspender auszutauschen und die Auswerteeinheit mit einem neuen Pumpspender weiterzuverwenden.

Es ist ein im Weiteren noch näher erläuterter Erfassungssensor zur Erfassung der Betätigung des Pumpspenders durch Niederdrücken des Betätigungsdrückers vorgesehen. Dieser erzeugt ein digitales oder analoges elektrisches Signal, welches von weiteren elektronischen Komponenten der Auswerteeinheit weiterverarbeitet und/oder weitergeleitet werden kann. Der Erfassungssensor ist vorzugsweise als Weg- oder Kraft- bzw. Drucksensor ausgebildet.

Er ist bei an dem Pumpspender angebrachter Auswerteeinheit vorzugsweise im Kraftfluss angeordnet. Dieser Kraftfluss verläuft bei üblicher Betätigung vom den Betätigungsdrücker betätigenden Finger des Benutzers zum Betätigungsdrücker, von dort zur Basis des Austragkopfes und dem Flüssigkeitsspeicher bis zu der Hand des Benutzers, die bei üblicher Bedienung den Flüssigkeitsspeicher umgreift.

Der Erfassungssensor ist vorzugsweise derart angeordnet, dass er die Verlagerung zwischen Betätigungsdrücker und Basis des Austragkopfes erfasst.

Erfindungsgemäß ist die Auswerteeinheit als externe Auswerteeinheit ausgebildet, die über ein Hauptsegment verfügt, welches lösbar ortsfest am Flüssigkeitsspeicher oder der Basis des Austragkopfes angebracht ist, und welche über ein Betätigungssegment verfügt, das gegenüber dem Hauptsegment beweglich ist und dessen Bewegung gegenüber dem Hauptsegment durch den Erfassungssensor erfassbar ist, wobei das Betätigungssegment derart an oder über dem Betätigungsdrücker angeordnet ist, dass beim Niederdrücken des Betätigungsdrückers gegenüber der Basis auch das Betätigungssegment gegenüber dem Hauptsegment verlagert wird.

Insbesondere handelt es sich beim Erfassungssensor um einen Kraftsensor oder Wegsensor, wobei für die meisten Anwendungsfälle eine digitale Auswertung ausreicht, so dass ein solcher Sensor insbesondere als einfacher Taster ausgebildet sein kann, der die Bewegung des Betätigungssegments gegenüber dem Hauptsegment erfasst.

Grundsätzlich sind aber auch andere Formen von Sensoren als Erfassungssensor verwendbar. So kann beispielsweise ein Erschütterungs- oder Beschleunigungssensor als Erfassungssensor genutzt werden, der mittelbar die Betätigung erfasst, beispielsweise indem Erschütterungen sensiert werden, die durch das Vorbeifahren des Betätigungsdrückers an einer oder mehreren Rastkanten verursacht wird. Das Vorhandensein eines Erschütterungs- oder Beschleunigungssensors in der Auswerteeinheit ist darüber hinaus auch für andere Funktionen von Vorteil. So kann damit beispielsweise ermittelt werden, ob der Pumpspender vor Verwendung ausreichend geschüttelt worden ist.

Die Auswerteeinheit kann auch über mindestens einen Sensor verfügen, der zur Erfassung der im Flüssigkeitsspeicher verbliebenen Flüssigkeitsmenge vorgesehen ist. Je nach Ausgestaltung kann auch der genannte Drucksensor bei der zweiten genannten Bauform im Ruhezustand des Sets erfassen, wie schwer der Pumpspender noch ist und hieraus auf die verbleibende Flüssigkeitsmenge rückschließen.

Darüber hinaus sind vielfältige weitere Sensoren denkbar, die zweckmäßig Teil der Auswerteeinheit sein können. So kann Sensorik vorgesehen sein, um zu erkennen, ob die Auswerteeinheit am Pumpspender angebracht ist. Auch ist es denkbar, dass ein für den Pumpspender eindeutiges Identifikationskennzeichen oder ein für die Flüssigkeit im Pumpspender spezifisches Identifikationskennzeichen vom Pumpspender ausgelesen werden, beispielsweise für RFID-Technologie. Weitere mögliche Sensoren sind Sensoren zur Erfassung der Flüssigkeitstemperatur und/oder der Umgebungstemperatur.

Die Auswerteeinheit kann derart ausgestaltet sein, dass die erfassten Daten unmittelbar in ihr gespeichert werden und/oder über eine Anzeigevorrichtung, insbesondere in Form von Leuchtdioden oder einem Display, dargestellt werden. Alternativ oder zusätzlich ist es bevorzugt, wenn die Auswerteeinheit über eine drahtlose Datenschnittstelle verfügt, insbesondere in Art einer Bluetooth-Schnittstelle, einer 4G-Schnittstelle oder einer 5G-Schnittstelle, über die erfasste Daten an einen Server oder ein separates Auswertegerät wie ein Mobiltelefon übermittelt werden.

Die Verwendung eines erfindungsgemäßen Sets gestattet es, an sich bekannte Pumpspender in einfacher Art und Weise derart zu ergänzen, dass die Handhabung durch den Benutzer erfassbar und weiterverarbeitbar ist. Hieraus ergeben sich verschiedene Nutzungsmöglichkeiten:
Aus Perspektive des Benutzers und insbesondere im Bereich kosmetischer Anwendungen ist die Möglichkeit der Datenauswertung von Vorteil, da der Benutzer seine Nutzungsart selbst kennenlernt und von einem auswertenden Programm Vorschläge hierzu erhalten kann. So kann ein solches Programm, welches insbesondere auf dem genannten externen Auswertegerät läuft, die Häufigkeit der Nutzung, die konkrete Art der Verwendung wie beispielsweise die Art des Drückens und ähnliches analysieren und Vorschläge für andere ergänzende Produkte wie insbesondere Kosmetikprodukte machen. Insbesondere wenn mehrere vom Benutzer verwendete Spender über Auswerteeinheiten der beschriebenen oder anderer Art verfügen, lassen sich zielgerichtet Vorschläge machen. Auch kann die Auswerteeinheit mittelbar oder unmittelbar den Füllstand eines Spenders prüfen und Nachbestellungen des entsprechenden Produktes automatisch oder halbautomatisch veranlassen.

Im Bereich pharmazeutischer Flüssigkeiten lässt sich die Einhalten von ärztlich vorgegebenen Anwendungszeiten kontrollieren. Die Daten können ggf. an den Arzt weitergegeben werden, so dass dieser einen Überblick über die Einhaltung der Zeiten erlangt.

Aus Perspektive des Herstellers der im Pumpspender gelagerter Flüssigkeit lassen sich wertvolle Daten zur typischen Nutzung ableiten. So kann erkannt werden, ob die Frequenz der Nutzung sich ändert oder wo die Verwendung des Pumpspenders üblicherweise stattfindet.

Der genannte Pumpspender ist ein im Wesentlichen üblicher Pumpspender. Es gibt keine zwingenden Anpassungen, die der Verwendung bekannter Pumpspender mit der beschriebenen Auswerteeinheit entgegenstehen. Dies erleichtert die Markteinführung des beschriebenen Systems. Nur wenn besondere Funktionalitäten gewünscht sind, wie beispielsweise die eindeutige Kennzeichnung eines Pumpspenders, insbesondere über ein RFID-Tag, bedarf es einer Anpassung des Pumpspenders.

Typische und fürsich genommen bekannte Merkmale, die ein Pumpspenderzur Verwendung in einem erfindungsgemäßen Set aufweist, sind die folgenden: Der Flüssigkeitsspeicher des Pumpspenders kann über eine Schnappverbindung oder eine Gewindeverbindung mit dem Austragkopf verbunden sein. Alternativ kann die Basis des Austragkopfes einstückig mit dem Flüssigkeitsspeicher ausgebildet sein. Der Flüssigkeitsspeicher ist vorzugsweise ein länglicher Körper, dessen Haupterstreckungsrichtung insbesondere vorzugsweise mit der Bewegungsrichtung des Betätigungsdrückers gegenüber der Basis übereinstimmt. Der Flüssigkeitsspeicher ist vorzugsweise im Wesentlichen kreiszylindrisch geformt, wobei er an einem oberen Ende üblicherweise mit einer Rastkante oder einem Gewinde zum Ankoppeln des Austragkopfes versehen ist. Der Flüssigkeitsspeicher ist im verkaufsfertigen Zustand mit einer Flüssigkeit befüllt, vorzugsweise mit einer kosmetischen oder einer pharmazeutischen Flüssigkeit, insbesondere mit einer Creme, einer Salbe oder einer Lotion.

Ein Pumpspender, der Teil eines erfindungsgemäßen Sets ist, kann an seiner Außenseite eine Beschriftung aufweisen, beispielsweise Markenzeichen, Hinweise auf Inhaltsstoffe der Flüssigkeit oder Verwendungshinweise.

Der Pumpspender weist gattungsgemäß eine Pumpeinrichtung auf, also eine Einrichtung, die beim Niederdrücken des Betätigungsdrückers eine Teilmenge der Flüssigkeit des Flüssigkeitsspeichers vom Flüssigkeitsspeicher isoliert und anschließend zur Austragöffnung leitet, wobei der für den Austrag vorgesehen Überdruck in der Pumpkammer vorzugsweise unmittelbar durch die Kraftbeaufschlagung des Betätigungsdrückers erzeugt wird. Insbesondere kann die Pumpeinrichtung eine Pumpkammer, sowie ein eingangsseitiges Einlassventil und ein ausgangsseitiges Auslassventil aufweisen, wobei das Einlassventil bei Unterdruck in der Pumpkammer gegenüber dem Flüssigkeitsspeicher öffnet und wobei das Auslassventil bei Überdruck in der Pumpkammer gegenüber einer umgebenden Atmosphäre öffnet. Die Pumpkammer ist volumetrisch veränderlich, wobei dies insbesondere vorzugsweise durch einen komprimierbaren Balg oder durch einen Pumpzylinder mit innenliegendem und beweglichem Pumpkolben erzielt wird.

Wenngleich Gestaltungen möglich sind, bei denen die Austragöffnung an der Basis des Austragkopfes vorgesehen ist, ist die bei Pumpspendern üblichere Gestaltung mit Austragöffnung am Betätigungsdrücker vorliegend bevorzugt. Bei einer solchen Gestaltung wird die Austragöffnung beim Niederdrücken des Betätigungsdrückers gegenüber der Basis des Austragkopfes ebenfalls niedergedrückt.

Das Funktionsprinzip basiert darauf, dass das Niederdrücken des Betätigungsdrückers gleichzeitig ein Niederdrücken des Betätigungssegments gegenüber dem zum Flüssigkeitsspeicher ortsfesten Hauptsegment verursacht, was zum Zwecke der Auswertung genutzt wird. Das Betätigungssegment kann hierfür seitlich am Betätigungsdrücker angebracht sein, so dass dieser weiterhin unmittelbar vom Benutzer niedergedrückt wird. Bevorzugt ist es allerdings, dass das Betätigungssegment bei der Betätigung zwischen dem Finger des Benutzers und dem Betätigungsdrücker angeordnet ist, so dass der Betätigungsdrücker nur mittelbar kraftbeaufschlagt wird.

Ein Set umfasst vorzugsweise eine Schutzkappe, die im aufgesetzten Zustand den Betätigungsdrücker und/oder die Austragöffnung überdeckt. Eine solche Schutzkappe wird üblicherweise zusammen mit bekannten Pumpspendern ausgeliefert. Sie verhindert, dass beispielsweise beim Transport in einer Tasche der Betätigungsdrücker versehentlich niedergedrückt wird.

Wenn eine solche Schutzkappe ausreichend groß ist, kann es im Einzelfall möglich sein, die Auswerteeinheit vollständig innerhalb des Aufnahmeraums der Schutzkappe unterzubringen.

Bevorzugt ist es allerdings, dass die Schutzkappe derart an den Austragkopf und das Hauptsegment der Auswerteeinheit angepasst ist, dass sie kraftschlüssig oder formschlüssig an den Austragkopf einerseits und an das Hauptsegment andererseits ankoppelbar ist. Dies gestattet es, eine Schutzkappe wahlweise bei Nichtverwendung der Auswerteeinheit unmittelbar am Austragkopf oder am Flüssigkeitsspeicher anzubringen und bei verwendeter Auswerteeinheit die Schutzkappe stattdessen an dieser anzubringen. Um ein Abfallen der Schutzkappe zu vermeiden, sollten diese einerseits und der Austragkopf sowie das Hauptsegment andererseits geeignet sein, eine stabile Kopplung zuzulassen. Dies kann beispielsweise durch entsprechende Rastelemente, durch ein Bajonett oder durch ein Gewinde erzielt werden.

Die Schutzkappe und das Hauptsegment der Auswerteeinheit können gleichartige Kopplungseinrichtungen aufweisen, die jeweils mit einer Kopplungseinrichtung am Austragkopf oder dem Flüssigkeitsspeicher koppelbar sind. Die Verwendung gleichartiger Kopplungseinrichtungen an der Schutzkappe und am Hauptsegment gestattet es, das Hauptsegment dort am Austragkopf oder am Flüssigkeitsspeicher anzubringen, wo bei fehlender Auswerteeinheit die Schutzkappe angebracht wird. Der Austragkopf bzw. der Flüssigkeitsspeicher müssen somit nicht in besonderer Art und Weise ausgestaltet sein, um eine Anbringung der Auswerteeinheit zu gestatten. Trotzdem kann durch geringfügige Variationen in den Maßen erreicht werden, dass eine am Austragkopf bzw. dem Flüssigkeitsspeicher angekoppelte Auswerteeinheit nur mit einem erhöhten Kraftaufwand lösbar ist, der höher als der üblicherweise zum Abnehmen der Schutzkappe erforderliche Kraftaufwand ist.

Das Hauptsegment einer erfindungsgemäßen Auswerteeinheit weist vorzugsweise einen Ringabschnitt auf, der einen Freibereich umgibt, durch den der Pumpspender bis in eine Kopplungsstellung eingeschoben werden kann. Die Ausgestaltung der Auswerteeinheit mit einem Ringabschnitt ist zum einen intuitiv, da durch den Freibereich des Ringabschnitts sehr einfach ersichtlich ist, wie die Auswerteeinheit am Pumpspender anzubringen ist. Zudem gestattet der Ringabschnitt einen besonders stabilen Halt und ist auch ästhetisch sehr gut an einem handelsüblichen Pumpspender zu integrieren.

Der Ringabschnitt ist vorzugsweise geschlossen. Es sind jedoch auch Gestaltungen denkbar, bei denen der Ring geschlitzt ist, beispielsweise um im montierten Zustand leicht aufgeweitet zu sein und dadurch eine Fixierung mittels starker Klemmkraft zu ermöglichen.

Am Ringabschnitt kann an einer Seite ein Aufnahmegehäuse für elektronische Komponenten und/oder eine Batterie vorgesehen sein. Ein solches asymmetrisch am Ringabschnitt angeordnetes Aufnahmegehäuse kann erforderlich werden, da es üblicherweise schwierig ist, die gewünschten elektronischen Komponenten einschließlich der Batterie alleine in einem Ringabschnitt vorzusehen. Insbesondere ist auch die Mechanik zur Führung des Betätigungssegments einfacher zu integrieren, wenn der Ringabschnitt in asymmetrischer Art und Weise durch ein daran angebrachtes oder angeformtes Aufnahmegehäuse ergänzt wird. Insbesondere ist ein solches Aufnahmegehäuse auch dann zweckmäßig, wenn die Auswerteeinheit selbst über ein Display verfügen soll. Ein solches ist üblicherweise im Ringabschnitt kaum gut lesbar unterzubringen.

Das Betätigungssegment kann gegenüber dem Hauptsegment auf verschiedene Arten verlagerbar sein. So ist beispielsweise eine Schwenkbeweglichkeit denkbar. Vorzugsweise ist das Betätigungssegment allerdings durch eine Schiebeführung translativ am Hauptsegment der Auswerteeinheit geführt. Die Schiebeführung kann je nach Ausgestaltung eine lineare Bewegung oder auch eine im leichten Bogen stattfindende Bewegung zulassen.

Die Schiebeführung ist sehr einfach realisierbar und insbesondere aufgrund der üblicherweise linearen Beweglichkeit des Betätigungsdrückers gegenüber der Basis des Austragkopfs geeignet, eine identische Beweglichkeit auch des Betätigungssegments gegenüber dem Hauptsegment zu ermöglichen.

Der Führungsabschnitt, der insbesondere vorzugsweise bei einer solchen Schiebeführung beim Niederdrücken des Betätigungssegments in ein Gehäuse des Hauptsegments eintaucht, ist vorzugsweise zur Erzielung einer erhöhten Stabilität ausgesteift. Dies kann insbesondere dadurch erreicht werden, dass der Führungsabschnitt einen gewölbten Querschnitt aufweist. Ein solcher gewölbter Querschnitt kann der üblicherweise runden Querschnittsform des Betätigungsdrückers folgend diesen partiell umgebend vorgesehen sein.

Das Betätigungssegment weist vorzugsweise eine Druckfläche auf, die von einer Seite über den Betätigungsdrücker hinüber ragt, so dass der Betätigungsdrücker durch Niederdrücken des Betätigungssegments mittelbar ebenfalls niedergedrückt wird.

Die genannte Druckfläche ist jene Fläche, die bei angebrachter Auswerteeinheit unmittelbar vom Benutzer kraftbeaufschlagt wird, so dass die Gefahr gering ist, dass versehentlich bei einer Betätigung des Betätigungsdrückers eine Betätigung des Betätigungssegments unterbleibt. Das Betätigungssegment ragt vorzugsweise von einer Seite über den Betätigungsdrücker und lässt im Übrigen den Betätigungsdrücker seitlich frei, so dass trotz des überragenden Betätigungsdrückers die Austragöffnung in Austragrichtung unbeeinflusst bleibt.

Die Auswerteeinheit weist vorzugsweise eine Getriebeeinrichtung auf, durch die die Verlagerung des Betätigungssegments gegenüber dem Hauptsegment in eine anders gerichtete oder anders limitierte Bewegung umgewandelt wird, welche dann zur Auslösung des Erfassungssensors genutzt wird.

Die Getriebeeinrichtung erleichtert es, die vom Betätigungssegment ausgehende Kraft derart umzulenken, dass ein hiervon beabstandeter Erfassungssensor, insbesondere ein Taster, ausgelöst wird. Insbesondere kann die Getriebeeinrichtung dahingehend eine limitierte Bewegung durchführen, dass die Verlagerung des Betätigungsdrückers in einer ersten Phase die Auslenkung der Getriebeeinrichtung bewirkt, diese dann jedoch nicht mehr weiter verlagert. Hierdurch wird die Gefahr verringert, dass ein Erfassungssensor, beispielsweise ein Taster, durch zu starkes Drücken des Betätigungssegments beschädigt wird. Insbesondere kann zu diesem Zweck die Getriebeeinrichtung über ein Kontaktglied verfügen, welches derart angeordnet ist, dass es bei Verlagerung des Betätigungssegments zunächst ebenfalls verlagert wird, und welches anschließend ohne Eigenverlagerung am fortgesetzt verlagerten Betätigungssegment abgleitet.

Wie eingangs bereits erläutert, kann die Auswertung der erfassten Daten unmittelbar an der Auswerteeinheit erfolgen, so dass diese keinerlei Schnittstellen braucht, um die Daten weiterzureichen. Bevorzugt ist jedoch eine Gestaltung, bei der es der Auswerteeinheit möglich ist, erfasste Daten an ein weiteres Gerät zu übersenden, weil dies tiefergehende Analysen und bessere visuelle Aufbereitung der erfassten Daten ermöglicht.

Grundsätzlich ist dabei denkbar, dass die Auswerteeinheit selbst mit dem Internet verbunden ist, beispielsweise über WLAN, 4G- oder 5G-Funknetzwerke. Sie kann dann ohne Zwischenschaltung eines weiteren aktiven Geräts des Benutzers die erfassten Daten an einen Zentralserver senden.

Bevorzugt ist es allerdings, wenn das Set mit Pumpspender und Auswerteeinheit zusätzlich ein mobiles Auswertegerät umfasst, insbesondere in Form eines Mobiltelefons des Benutzers, welches über eine drahtlose Schnittstelle, insbesondere die genannte Bluetooth-Schnittstelle, mit der Auswerteeinheit verbunden ist. Auf diesem mobilen Auswertegerät können Programme zur Auswertung der erfassten Daten laufen. Auch können weitere Sensoren des Auswertegeräts herangezogen werden, um die Daten der Auswerteeinheit zu ergänzen. Beispielsweise ist eine globale Lokalisierung, wie sie zum Beispiel über GPS möglich ist, mit einem vergleichsweise großen technischen Aufwand verbunden, so dass es von Vorteil sein kann, die Auswerteeinheit von einer entsprechenden technischen Gestaltung freizuhalten und stattdessen das mobile Auswertegerät, also insbesondere ein Mobiltelefon mit einem Programm (App), zur Erfassung der Position heranzuziehen. Allerdings kann es durchaus zweckmäßig sein, die Auswerteeinheit selbst mit einer Einrichtung zur globalen Lokalisierung zu versehen, da nicht immer gewährleistet sein muss, dass das mobile Auswertegerät am gleichen Ort wie die Auswerteeinheit ist.

Zu den Daten, die die Auswerteeinheit oder das mobile Auswertegerät speichern oder auswerten, können die unmittelbar die Betätigung betreffenden Daten wie Zeit und Ort gehören. Es können aber auch Charaktermerkmale der Betätigung, wie beispielsweise die Betätigungsgeschwindigkeit oder die Hublänge ausgewertet werden. Auch die Auswertung von Umgebungsparametern, wie beispielsweise der Temperatur, kann zweckmäßig sein. Die Daten werden vorzugsweise entweder direkt von der Auswerteeinheit oder von dem mobilen Auswertegerät an einen Zentralserver versendet, der ergänzende Analysen, auch über mehrere Benutzer hinweg, ermöglicht.

Das Set umfasst vorzugsweise ein mobiles Auswertegerät, insbesondere in Form eines Mobiltelefons, welches über eine drahtlose Schnittstelle mit der Auswerteeinheit verbunden ist.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert sind.
Fig. 1 zeigt einen üblichen Pumpspender, wie er aus dem Stand der Technik bekannt ist.
Die Fig. 2 und 3 zeigen zum Pumpspender der Fig. 1 in geschnittener Darstellung die Funktionsweise bei Betätigung.
Fig. 4 zeigt ein erfindungsgemäßes Set mit dem Pumpspender der Fig. 1 bis 3 sowie einer daran angebrachten Auswerteeinheit.
Fig. 5 zeigt in geschnittener Darstellung die Einzelkomponenten des Sets der Fig. 4 und die Art der Montage.
Fig. 6 zeigt das Set gemäß Fig. 4 in geschnittener Darstellung nach abgeschlossener Montage.
Die Fig. 7 und 8 zeigen zum Set der Fig. 4 in geschnittener Darstellung die Funktionsweise bei Betätigung.

### DETAILLIERTE BESCHREIBUNG DES AUSFÜHRUNGSBEISPIELS

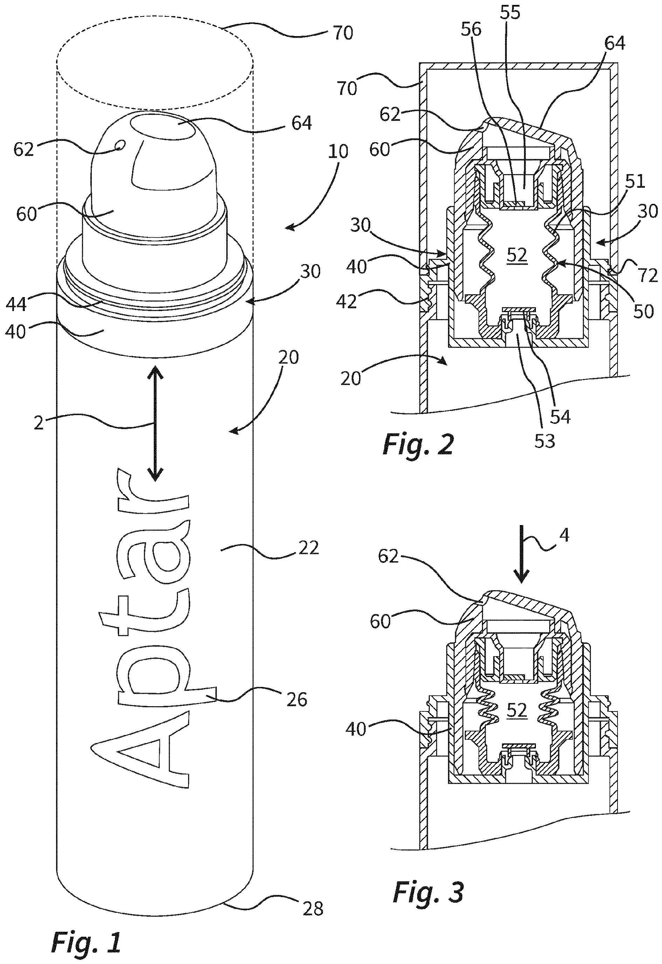

Die Fig. 1 bis 3 zeigen einen im Wesentlichen herkömmlichen Pumpspender, wie er Teil eines erfindungsgemäßen Sets wird.

Bezug nehmend auf Fig. 1 ist zu erkennen, dass der Pumpspender 10 über einen als nach oben offener Behälter ausgebildeten Flüssigkeitsspeicher 20 verfügt, auf dessen Mantelwandung 22 eine Beschriftung 26 vorgesehen ist. An den Flüssigkeitsspeicher ist mittels einer Kopplungseinrichtung 42 in Art eines Gewindes ein Austragkopf 30 angekoppelt. Dieser verfügt über einen in Richtung der Haupterstreckungsrichtung 2 des Pumpspenders niederdrückbaren Betätigungsdrücker 60, der bestimmungsgemäß manuell durch Kraftbeaufschlagung im Bereich einer Betätigungsfläche 64 niedergedrückt wird, um hierdurch Flüssigkeit aus dem Flüssigkeitsspeicher 20 durch eine am Betätigungsdrücker vorgesehene Austragöffnung 62 auszutragen. Im nicht benutzten Zustand kann der Austragkopf durch eine Schutzkappe 70 überdeckt werden, die im Bereich einer Rastkante 44 an der Basis 40 des Austragkopfs rastend befestigt ist.

Wie der Fig. 2 zu entnehmen ist, ist innerhalb des Austragkopfs 30 eine Pumpeinrichtung 50 vorgesehen. Diese umfasst eine durch Niederdrücken des Betätigungsdrückers 60 volumetrisch verkleinerbare Pumpkammer 52, die über einen Einlasskanal 53 mit dem Flüssigkeitsspeicher 20 und über einen Auslasskanal 55 mit der Austragöffnung 62 verbunden ist. Sowohl der Einlasskanal 53 als auch der Auslasskanal 55 verfügen jeweils über ein Ventil 54, 56. Das Einlassventil 54 öffnet bei Unterdruck in der Pumpkammer 52 gegenüber dem Flüssigkeitsspeicher 20 und somit bei einer nach oben gerichteten Rückstellbewegung des Betätigungsdrückers 60. In dieser Phase ist das Auslassventil 56 geschlossen, da es nur bei Überdruck in der Pumpkammer 52 gegenüber einer umgebenden Atmosphäre öffnet und anderenfalls schließt. Beim Niederdrücken des Betätigungsdrückers 60 hingegen öffnet sich das Auslassventil 56, während das Einlassventil 54 geschlossen ist. Auf diese Art und Weise ist in der in Fig. 3 ersichtlichen Weise möglich, Flüssigkeit aus der Pumpkammer 52 durch Niederdrücken des Betätigungsdrückers 60 in Betätigungsrichtung 4 zu bewirken. Beim vorliegenden Ausführungsbeispiel ist die Pumpeinrichtung 50 als Balgpumpeinrichtung ausgebildet und weist daher einen Balgkörper 51 auf, der die Pumpkammer 52 umgibt. Andere mögliche Bauweisen sehen beispielsweise eine Kolbenpumpe vor, bei der beim Niederdrücken des Betätigungsdrückers 60 ein Kolben innerhalb eines Zylinders zur Verkleinerung der Pumpkammer 52 verlagert wird.

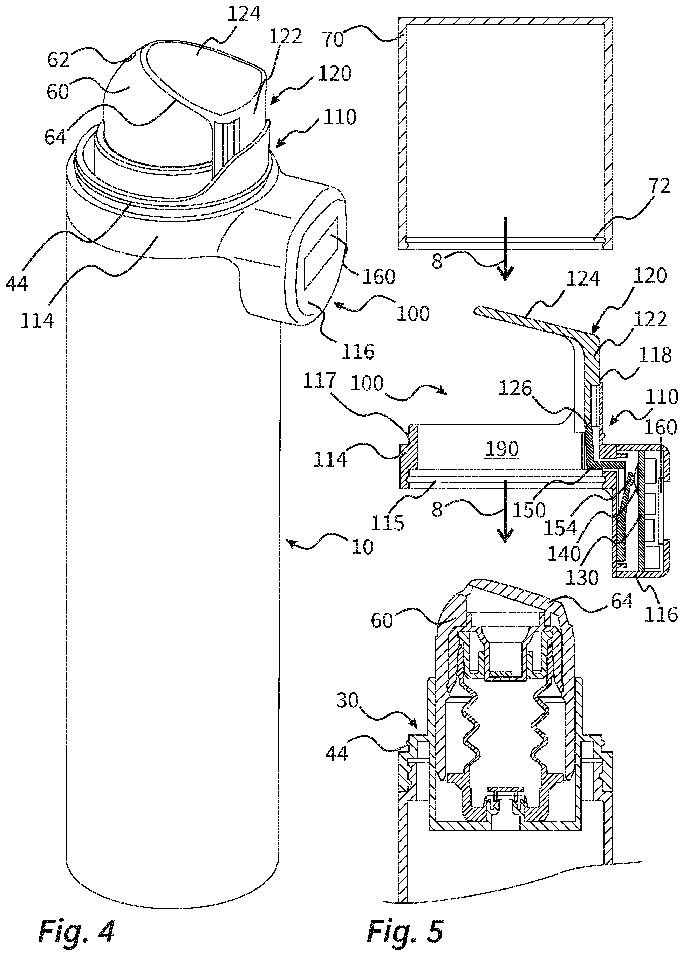

Die Fig. 4 bis 8 zeigen ein erfindungsgemäßes Set.

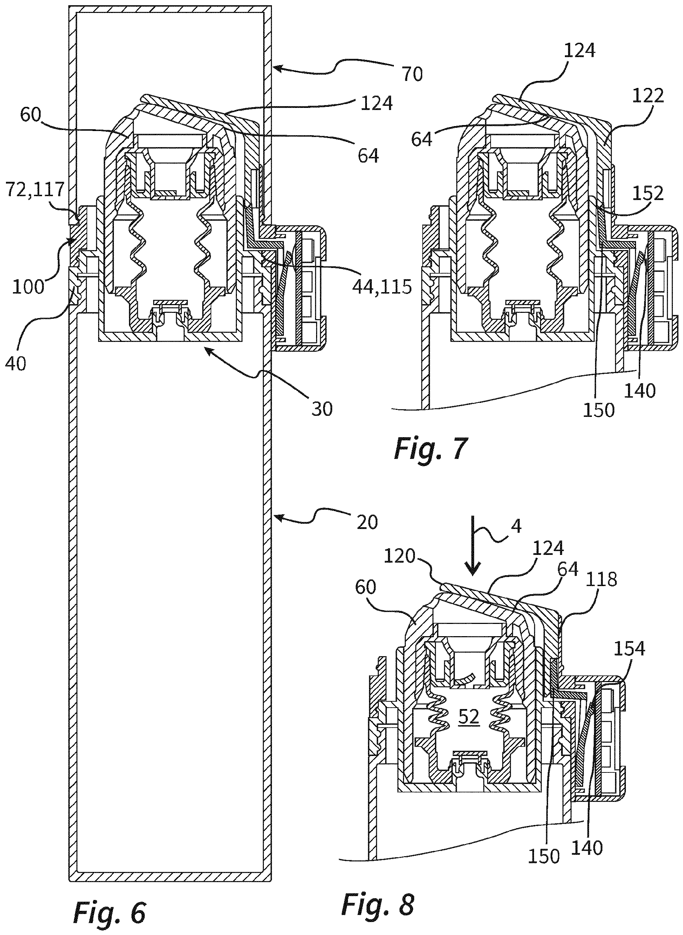

In Fig. 4 ist ebenso wie in Fig. 6 der montierte Zustand dargestellt. Es ist zu ersehen, dass der zuvor schon beschriebene Pumpspender 10 ergänzt ist durch eine Auswerteeinheit 100. Diese Auswerteeinheit 100 wird anhand der Vormontagesituation von Fig. 5 erläutert, in der sie geschnitten dargestellt ist.

Die Auswerteeinheit 100 verfügt über einen Ringabschnitt 114, der an einer Seite in ein Aufnahmegehäuse 116 zur Aufnahme elektronischer Komponenten übergeht. Gemeinsam bilden der Ringabschnitt 114 und das Aufnahmegehäuse 116 die wesentlichen Teile eines Hauptsegments 110 der Auswerteeinheit 100. Das zweite von außen ersichtliche Element der Auswerteeinheit 100 ist ein Betätigungssegment 120, welches in etwa L-förmig geformt an der Oberseite der Auswerteeinheit 100 vorgesehen ist und mittels einer vertikalen Schiebeführung 118 translativ gegenüber dem Hauptsegment 110 verlagerbar ist. Dies ist in im Weiteren noch erläuterter Weise geeignet, ein Getriebebauteil 150 zu verschwenken, welches seinerseits einen Taster 140 betätigt.

Der Taster 140 ist Teil einer Sensoreinheit 130 im Aufnahmegehäuse 116. Die Sensoreinheit 130 weist darüber hinaus in nicht näher bezeichneter Weise Funkelemente für Bluetooth, WLAN und/oder Mobilfunk (3G, 4G, 5G) auf. Weiterhin umfasst ist eine Batterie als Stromversorgung sowie weitere Sensoren, wie insbesondere ein Temperatursensor, ein GPS-Chip und ein Beschleunigungssensor. Beim vorliegenden Ausführungsbeispiel ist weiterhin noch ein Display 160 vorgesehen, welches je nach Verwendungszweck jedoch auch entfallen kann.

Weiterhin Bezug nehmend auf Fig. 5 ist ersichtlich, wie die Montage erfolgt. An der Innenseite des Ringabschnitts 114 des Hauptsegments 110 ist eine Kopplungseinrichtung in Art einer Nut 115 vorgesehen. Diese ist so bemessen, dass sie an die Rastkante 44 der Basis 40 des Austragkopfs ankoppelbar ist, wobei die so gefügte Verbindung bewusst recht schwergängig ist, damit die Auswerteeinheit nicht versehentlich zusammen mit der Kappe abgezogen wird. Der Ringabschnitt 114 des Hauptsegments 110 verfügt weiterhin über eine Rastkante 117, die bezüglich ihrer Ausgestaltung in etwa jener der Rastkante 44 entspricht, so dass die Schutzkappe 70 mit der an ihr vorgesehenen Rastnut 72 hier verrastet werden kann.

Mit anderen Worten wird bei der Montage eines Sets gemäß den Fig. 4 bis 8 die Auswerteeinheit 100 dort am Austragkopf 30 befestigt, wo anderenfalls die Schutzkappe 70 befestigt würde. Die Schutzkappe 70 ihrerseits wird an der Rastkante 117 derAuswerteeinheit 100 befestigt.

Fig. 6 zeigt in geschnittener Darstellung den montierten Zustand.

Anhand der Fig. 7 und 8 wird die Bedienung verdeutlicht. Das Betätigungssegment 120 der Auswerteeinheit 100 ragt über die Betätigungsfläche 64 des Betätigungsdrückers 60 hinweg, so dass eine Kraftbeaufschlagung des Betätigungsdrückers 60 intuitiv und offensichtlich für den Benutzer durch Niederdrücken des Betätigungssegments 120 erfolgt. Hierfür ist an der Oberseite des Betätigungssegments 120 eine Druckfläche 124 vorgesehen. Wenn in der in Fig. 8 verdeutlichten Weise das Betätigungssegment 120 in Betätigungsrichtung 4 niedergedrückt wird, so führt dies auch zu einem Niederdrücken des Betätigungsdrückers 60 und demzufolge zu einem Austrag von Flüssigkeit.

Gleichzeitig jedoch drückt eine untere Kante 126 des Betätigungssegments 120 das Getriebebauteil 150 zur Seite, wie Fig. 8 zeigt. Dieses schwenkt dabei um eine am unteren Ende des Getriebebauteils 150 vorgesehene Schwenkachse, so dass ein Ausleger 154 den Taster 140 auslöst und somit die genannte Sensoreinheit 130 der Auswerteeinheit 100 den erfolgten Austragvorgang erkennen lässt. Das Sensormodul kann die Betätigung mit Zeitstempel, Ortsangabe und/oder Daten der anderen Sensoren gemeinsam abspeichern und/oder sie drahtlos an einem Empfänger senden.

Ein fortgesetztes Niederdrücken des Betätigungssegments 120 führt nicht zu einem weiteren Auslenken des Getriebebauteils 150, da dieses dann am Betätigungssegment 120 entlanggleitet. Es ist daher nicht zu befürchten, dass eine zu weite Verlagerung des Betätigungssegments 120 anderweitige Komponenten der Auswerteeinheit 100 beschädigt.

## Patentansprüche

1. Set aus einem Pumpspender (10) zum Austrag von pharmazeutischen oder kosmetischen Flüssigkeiten und einer Auswerteeinheit (100) mit den folgenden Merkmalen:
a. der Pumpspender (10) verfügt über einen Flüssigkeitsspeicher (20), an dessen oberen Ende ein Austragkopf (30) angebracht ist, und
b. der Austragkopf (30) verfügt über eine Basis (40), die am Flüssigkeitsspeicher (20) befestigt ist, sowie über einen Betätigungsdrücker (60), der zum Zwecke des Austrags gegenüber der Basis (40) niederdrückbar ist, und
c. der Austragkopf (30) verfügt über eine Pumpeinrichtung (50), die durch Niederdrücken des Betätigungsdrückers (60) betätigt werden kann und die bei Betätigung Flüssigkeit von einem Einlasskanal (53), der mit dem Flüssigkeitsspeicher (20) verbunden ist, zu einer am Austragkopf (30) vorgesehenen Austragöffnung (62) fördert, und
d. die Auswerteeinheit (100) ist zur Erfassung der Betätigung der Pumpeinrichtung (50) ausgebildet, wobei die Auswerteeinheit (100) als externe Auswerteeinheit (100) ausgebildet ist, die über ein Hauptsegment (110) verfügt, welches lösbar ortsfest am Flüssigkeitsspeicher (20) oder der Basis (40) des Austragkopfes (30) angebracht ist, und welche über ein Betätigungssegment (120) verfügt, das gegenüber dem Hauptsegment (110) beweglich ist und dessen Bewegung gegenüber dem Hauptsegment (110) durch einen Erfassungssensor (140) erfassbar ist, wobei das Betätigungssegment (120) derart an oder über dem Betätigungsdrücker (60) angeordnet ist, dass beim Niederdrücken des Betätigungsdrückers (60) gegenüber der Basis (40) auch das Betätigungssegment (120) gegenüber dem Hauptsegment (110) verlagert wird

2. Set nach Anspruch 1 mit dem folgenden zusätzlichen Merkmal:
a. das Set umfasst eine Schutzkappe (70), die im aufgesetzten Zustand den Betätigungsdrücker 60) und/oder die Austragöffnung (62) überdeckt.

3. Set nach Anspruch 2 mit dem folgenden zusätzlichen Merkmal:
a. die Schutzkappe (70) ist derart an den Austragkopf (30) und das Hauptsegment (110) der Auswerteeinheit (100) angepasst, dass sie kraftschlüssig oder formschlüssig an den Austragkopf (30) und das Hauptsegment (110) ankoppelbar ist,
vorzugsweise mit mindestens einem der folgenden weiteren Merkmale:
b. die Schutzkappe (70) und das Hauptsegment (110) der Auswerteeinheit (100) weisen gleichartige Kopplungseinrichtungen (72, 112) auf, die jeweils mit einer Kopplungseinrichtung (42) am Austragkopf (30) oder dem Flüssigkeitsspeicher (20) koppelbar sind und/oder
c. die Schutzkappe (70) ist zur rastenden Kopplung am Hauptsegment (110) der Auswerteeinheit (100) ausgebildet und das Hauptsegment (110) ist zur rastenden Kopplung am Austragkopf (30) oder dem Flüssigkeitsspeicher (20) ausgebildet, wobei eine geringere Zugkraft zum Lösen der rastenden Kopplung zwischen Schutzkappe (70) und Hauptsegment (110) vorgesehen ist.

4. Set nach einem der vorstehenden Ansprüche mit dem folgenden zusätzlichen Merkmal:
a. das Hauptsegment (110) der Auswerteeinheit (100) weist einen Ringabschnitt (114) auf, der einen Freibereich (190) umgibt, durch den der Pumpspender (10) bis in eine Kopplungsstellung eingeschoben werden kann,
vorzugsweise mit dem folgenden weiteren Merkmal:
b. am Ringabschnitt (114) ist an einer Seite ein Aufnahmegehäuse (116) für elektronische Komponenten und/oder eine Batterie vorgesehen.

5. Set nach einem der vorstehenden Ansprüche mit dem folgenden zusätzlichen Merkmal:
a. das Betätigungssegment (120) ist durch eine Schiebeführung (118) translativ am Hauptsegment (110) der Auswerteeinheit (100) geführt.

6. Set nach Anspruch 5 mit dem folgenden zusätzlichen Merkmal:
a. das Betätigungssegment (120) weist einen Führungsabschnitt (122) der Schiebeführung (118) auf, der einen gewölbten Querschnitt aufweist, mit dem der Führungsabschnitt (122) den Betätigungsdrücker partiell umgibt.

7. Set nach einem der vorstehenden Ansprüche mit dem folgenden zusätzlichen Merkmal:
a. das Betätigungssegment (120) weist eine Druckfläche (124) auf, die von einer Seite über den Betätigungsdrücker (60) hinüber ragt, so dass der Betätigungsdrücker (60) durch Niederdrücken des Betätigungssegments (120) mittelbar ebenfalls niedergedrückt wird,

8. Set nach einem der vorstehenden Ansprüche mit dem folgenden zusätzlichen Merkmal:
a. die Auswerteeinheit (100) weist eine Getriebeeinrichtung (150) auf, durch die die Verlagerung des Betätigungssegments gegenüber dem Hauptsegment in eine anders gerichtete oder anders limitierte Bewegung umgewandelt wird, welche dann zur Auslösung des Erfassungssensors (140) genutzt wird.

9. Set nach Anspruch 6 mit dem folgenden zusätzlichen Merkmal:
a. die Getriebeeinrichtung verfügt über ein Kontaktglied, welches derart angeordnet ist, dass es bei Verlagerung des Betätigungssegments zunächst ebenfalls verlagert wird und anschließend ohne Eigenverlagerung am fortgesetzt verlagerten Betätigungssegment abgleitet.

10. Set nach einem der vorstehenden Ansprüche mit mindestens einem der folgenden zusätzlichen Merkmale:
a. der Flüssigkeitsspeicher (20) des Pumpspenders (10) ist über eine Schnappverbindung oder eine Gewindeverbindung mit dem Austragkopf (30) verbunden oder die Basis des Austragkopfes ist einstückig mit dem Flüssigkeitsspeicher ausgebildet, und/oder
b. der Flüssigkeitsspeicher (20) ist im Wesentlichen kreiszylindrisch geformt, und/oder
c. der Flüssigkeitsspeicher (20) ist im Bereich einer Mantelwandung (22) außenseitig mit einer Beschriftung (26) versehen, und/oder
d. die Pumpeinrichtung (50) weist eine Pumpkammer (52), ein eingangsseitiges Einlassventil (54) und ein ausgangsseitiges Auslassventil (56) auf, wobei das Einlassventil (54) bei Unterdruck in der Pumpkammer (52) gegenüber dem Flüssigkeitsspeicher (20) öffnet und wobei das Auslassventil (56) bei Überdruck in der Pumpkammer (52) gegenüber einer umgebenden Atmosphäre öffnet, und/oder
e. die Austragöffnung (62) ist am Betätigungsdrücker (60) angebracht, so dass sie beim Niederdrücken des Betätigungsdrückers (60) gegenüber der Basis (40) des Austragkopfes (30) ebenfalls niedergedrückt wird, und/oder
f. der Flüssigkeitsspeicher ist mit einer kosmetischen oder einer pharmazeutischen Flüssigkeit befüllt, insbesondere mit einer Creme, einer Salbe oder einer Lotion.

11. Set nach einem der vorstehenden Ansprüche mit dem folgenden zusätzlichen Merkmal:
a. das Set umfasst ein mobiles Auswertegerät (300), insbesondere in Form eines Mobiltelefons, welches über eine drahtlose Schnittstelle mit der Auswerteeinheit verbunden ist,
vorzugsweise mit mindestens einem der folgenden weiteren Merkmale:
b. die drahtlose Datenschnittstelle ist eine Bluetooth-Schnittstelle, und/oder
c. die Auswerteeinheit (100) verfügt über einen Speicher, in dem vom Erfassungssensor erfasste Daten abgelegt werden, um später bei bestehender Funkverbindung an das mobile Auswertegerät (300) oder einen Zentralserver (400) übertragen zu werden.

12. Set nach einem der vorstehenden Ansprüche mit mindestens einem der folgenden zusätzlichen Merkmale:
a. die Auswerteeinheit (100) verfügt über mindestens einen Sensor in Art eines Drucksensors, Kraftsensors oder Wegsensors (140), insbesondere in Form eines Tasters (140), und/oder
b. die Auswerteeinheit (100) verfügt über mindestens einen Sensor, der zur Erfassung der im Flüssigkeitsspeicher verbliebenen Flüssigkeitsmenge vorgesehen ist, und/oder
c. die Auswerteeinheit (100) verfügt über mindestens einen Beschleunigungssensor, und/oder
d. die Auswerteeinheit (100) oder das mobile Auswertegerät (300) verfügt über mindestens einen Sensor in Art einer globalen Positionserfassungseinrichtung, und/oder
e. die Auswerteeinheit (100) oder das mobile Auswertegerät (300) sind dafür ausgebildet, zu jeder durch den Erfassungssensor erfassten Betätigung die Zeit, den Ort, Charaktermerkmale der Betätigung oder Umgebungsparameter abzuspeichern und/oder über das Internet an einen Zentralserver zu versenden, und/oder
f. die Auswerteeinheit verfügt über eine Anzeigeeinrichtung, insbesondere in Form von Leuchtdioden oder einem Display (160).
